(19) Europäisches Patentamt
European Patent Office
Office européen des brevets

(11) **EP 0 976 414 A2**

(12) **EUROPÄISCHE PATENTANMELDUNG**

(43) Veröffentlichungstag:
02.02.2000 Patentblatt 2000/05

(51) Int. Cl.[7]: **A61M 1/38**

(21) Anmeldenummer: **99104790.3**

(22) Anmeldetag: **10.03.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **10.03.1998 DE 19810195**

(71) Anmelder:
**Salinger, Reinhard Dr. med.**
**90542 Eckental (DE)**

(72) Erfinder:
**Salinger, Reinhard Dr. med.**
**90542 Eckental (DE)**

(74) Vertreter:
**Schneck, Herbert, Dipl.-Phys., Dr. et al**
**Rau, Schneck & Hübner**
**Patentanwälte**
**Königstrasse 2**
**90402 Nürnberg (DE)**

(54) **Verfahren und Vorrichtung zur Sammlung und Weiterverarbeitung von spezifischen Blutkomponenten bei kontunierlichen laminaren Strömungsverhältnissen**

(57) Bei einem Verfahren zur Sammlung von spezifischen Blutkomponenten bei kontinuierlichen, laminaren Strömungsverhältnissen, wobei dem Patienten mittels einer ersten Leitung Blut entnommen und einer der Separation der Blutkomponenten dienenden Zentrifuge zugeführt und über eine Rückführleitung dem Patienten Blut mit der Entnahme-Zuflußrate zurückgeführt wird, wobei die Separation bestimmt wird durch den Geometriefaktor der Zentrifuge, durch die Rotationsgeschwindigkeit der Zentrifuge und die Positionierung der Entnahmeleitung an der Zentrifuge, und wobei charakteristisch für die Separation einzelner bestimmter Blutkomponenten ein Trennungsoptimierungskoeffizient $T_{OK}$ ist, ist vorgesehen, daß zur Sammlung von Blutstammzellen bzw. mononuklearen Zellen der Trennungsoptimierungskoeffizient $T_{OK}$ zwischen 140 und 190, vorzugsweise bei ca. 176 liegt, und/oder daß bei Sammlung von Thrombozyten bei Blutflußgeschwindigkeiten zwischen 30 und 100 ml/min der Trennungsoptimierungskoeffizient zwischen 600 und 700, vorzugsweise bei ca. 649 liegt.

EP 0 976 414 A2

**Beschreibung**

Die Erfindung richtet sich auf eine Vorrichtung gemäß dem Oberbegriff von Anspruch 1, wie sie beispielsweise aus DE 38 28 120 C2, DE 38 28 618 C2 und EP 0 654 277 A1 bekannt ist.

Für die semiqualitative Sammlung von spezifischen Blutkomponenten, Thrombozyten, Monozyten bzw. dendritischer Zellen oder peripheren Blutstammzellen (CD34+-Zellen) bzw. Sammlung der genannten Zellen aus Knochenmarksblut werden Apheresesysteme auf der Grundlage der Zentrifugalmethode genutzt. Die physikalischen Eigenschaften einer Zentrifuge werden zur Trennung von Blutkomponenten aus zweierlei Gründen eingesetzt, einmal um die Sedimentationsgeschwindigkeit von „Makromolekülen" in den Bereich der Messbarkeit [Konzentrierung] anzuheben und zum anderen um eine gute Trennung der entsprechenden Komponenten des Gemisches (Blut) zu erreichen. Beides ist möglich, weil anstelle der Schwerkraft die ganz wesentlich vergrößerte Zentrifugalkraft ausgenutzt werden kann. Die größte Bedeutung hat die Zentrifuge wegen der deutlich vergrößerten Sedimentationsgeschwindigkeit. Grundsätzlich erhält man aber keine völlig scharfe Trennung der Komponenten, sondern für jede Komponente kommt es zu einer Verteilung der Anzahldichte, die stets ihr Maximum beim größtmöglichen Abstand von der Drehachse hat. Dabei ist zu berücksichtigen, daß das Optimum für die Sammlung die jeweiligen Komponenten in einem ausgewogenen Verhältnis der Abstände Sediment-ationsgeschwindigkeiten, ihrer Anzahldichten sowie der Kompressibilität der Komponenten abhängt. Inkompressible Medien, wie Blut, haben eine konstante Dichte. Um eine gute Trennung der genannten Blutkomponenten des Blutgemisches zu erreichen sind folgende Punkte zu berücksichtigen:

**aa)** Handelt es sich bei der eingesetzten Methode um ein kontinuierliches oder diskontinuierliches Verfahren.

**ab)** Bei kontinuierlichen Verfahren wird das Blutkomponenten-Gemisch mit einer vorher definierten Geschwindigkeit ml/min einer Person oder einem Knochenmarksblut-Sammelbeutel, mittels eines Katheders (zentral oder peripher) oder durch Konnektierung des Knochenmarkblut-Sammelbeutels mit dem sterilen Apherese-Schlauchsystem entnommen und der Zentrifugen - Apheresemaschine zugeführt. Innerhalb der Apheresemaschine wird das Zellkomponenten-Gemisch den zur Trennung der Komponenten notwendigen biologisch-physikalischen Gesetzen unterzogen und dem Spender/Knochenmarksblut-Sammelbeutel über einen Rückführungsschlauch mit der gleichen Entnahme—Volumen-Geschwindigkeit ml/min zurückgeführt. In der Apheresemaschinen -Trennkammer herrschen folglich die gleichen Geschwindigkeiten.

**b)** Herrschen während des Trennvorganges laminare oder turbulente Strömungsverhältnisse vor Nur auf das laminare Strömen von Flüssigkeiten mit konstanter Viskosität kann das Gesetz, von Hagen — Poiseuille angewendet werden. Es ist deshalb notwendig zuerst zu klären ob laminare Strömungsverhältnisse während des Trennvorganges vorherrschen. Für diese Klärung hilft die Reynolds-Zahl R$e$.

**c) Definition der Reynolds-Zahl R$e$**

Wenn die Strömungsgeschwindigkeit einer Flüssigkeit eine gewisse Grenze überschreitet, dann geht die laminare Strömung in eine turbulente Strömung über. Diese kritische Geschwindigkeit hängt von der Dichte und der Viskosität der Flüssigkeit sowie vom „Radius" der Röhre ab. Eine wichtige Kennzahl zur Charakterisierung von Flüssigkeitsströmen ist die Reynolds-Zahl R$e$, die durch

$$\mathrm{R}e = \frac{2r\sigma\upsilon}{\eta}$$

definiert ist, wobei $\upsilon$ die mittlere Strömungsgeschwindigkeit der Flüssigkeit ist. Experimente haben gezeigt, daß die Strömung von Flüssigkeiten mit einer Reynold-Zahl von bis zu 2000 laminar und für Werte über 3000 turbulent ist. Für Werte zwischen diesen beiden Grenzen ist die Strömung instabil und kann von einem Typ in den anderen übergehen.

$$\mathrm{R}e = \frac{2*(...m)* (1060 \text{ kg/m}^3)*\text{m/s}}{4*10^{-3} \text{ Pa*s}}$$

**d) Reynoldsche Zahlen von Blut in Abhängigkeit def. Strömungsgeschwindigkeiten**

Tabelle 1

| GBV | v = m/s | R*e* | GBV | v = m/s | R*e* |
|---|---|---|---|---|---|
| ml/min | | | ml/min | | |
| 174 | 0,01465 | 61 | 92 | 0,00774 | 32 |
| 164 | 0,01379 | 58 | 82 | 0,00689 | 29 |
| 154 | 0,01295 | 54 | 72 | 0,00605 | 25 |
| 144 | 0,01211 | 51 | 62 | 0,00521 | 22 |
| 133 | 0,01118 | 47 | 51 | 0,00429 | 18 |
| 123 | 0,01034 | 43 | 41 | 0,00345 | 14 |
| 113 | 0,00950 | 40 | 31 | 0,00261 | 11 |
| 103 | 0,00866 | 36 | | | |

Zum Vergleich: die Strömungsgeschwindigkeit des Blutes beim Ausstoß aus der linken Herzkammer (bei 75ml pro Kontraktion) in die Aorta beträgt ca. $v = 1$ m/s, d.h. eine R*e* = 5000. Die Strömung in der Aorta ist somit eher turbulent.

Tabelle 2

| Reynoldsche Zahlen der einzelnen zellulären Blutkomponenten | | | | | | |
|---|---|---|---|---|---|---|
| GBV ml/min | Thrombo | Lympho | CD34+ | Mono | PNC | Ery |
| 154 | 53 | 54 | 54 | 54 | 56 | 56 |
| 144 | 50 | 50 | 51 | 51 | 52 | 52 |
| 133 | 46 | 47 | 47 | 47 | 48 | 48 |
| 123 | 42 | 43 | 43 | 43 | 44 | 45 |
| 113 | 39 | 40 | 40 | 40 | 41 | 41 |
| 103 | 36 | 36 | 36 | 36 | 37 | 37 |
| 92 | 32 | 32 | 32 | 32 | 33 | 33 |
| 82 | 28 | 29 | 29 | 29 | 30 | 30 |
| 72 | 25 | 25 | 25 | 25 | 26 | 26 |
| 62 | 21 | 22 | 22 | 22 | 22 | 23 |
| 51 | 18 | 18 | 18 | 18 | 18 | 19 |
| 41 | 14 | 14 | 14 | 14 | 15 | 15 |

**e) Gesetz von Hagen — Poiseuille**

Bedeutung des von Hagen-Poiseuillen Gesetzes für Blut.

$$\delta p = \frac{8\eta |}{r\pi^4} V$$

Blut ist eine komplexe Flüssigkeit bestehend aus festen Bestandteilen mit verschiedenen Formen, die im Blutplasma suspendiert sind. Die roten, scheibenförmigen Blutkörperchen, sind bei niedrigen Geschwindigkeiten zufällig orientiert, bei hohen Geschwindigkeiten orientieren sie sich um das Fließen zu erleichtern. Aus diesem Grund nimmt die Viskosität von Blut ab, wenn die Strömungsgeschwindigkeit zunimmt, und das Gesetz von Hagen-Poiseuille ist streng genommen nicht mehr gültig. Trotzdem ist es eine gute Näherung, die Gesetzmäßigkeiten des Blutflusses qualitativ verstehen hilft.

Wenn die Strömungsgeschwindigkeit einer Flüssigkeit eine gewisse Grenze überschreitet, dann geht die laminare Strömung in eine turbulente Strömung über Diese kritische Geschwindigkeit hängt von der Dichte und der Viskosität der Flüssigkeit sowie vom „Radius" der Röhre/Kanals ab.

### f) Viskositätswiderstand = Reibungswiderstand

Beim Sinken eines Körpers in einer Flüssigkeit müssen mehrere Kräfte berücksichtigt werden. Es wirken zum einen die Gravitationskraft $F_{Gra}$ und der entgegengerichtete Auftrieb $F_{Auf}$. Für die wirksame Differenz beider Kräfte gilt:

$$F_{Gra} \longrightarrow F_{Auf} = 4/3 * \pi * r^3 * (\sigma - \sigma_{fl}) * g$$

Die Masse **m** der Kugel bzw. des kugeligen Elementes und die Masse $m_{fl}$ der verdrängten Flüssigkeitsmenge. werden durch das Volumen und die zugehörigen Dichten $\sigma$ und $\sigma_{fl}$ ausgedrückt. Wenn nur diese resultierende Kraft wirken würde, ergebe sich eine nach unten beschleunigte Bewegung der Kugel. Dies geschieht auch. Es stellt sich aber schließlich eine gleichförmige Sinkgeschwindigkeit ein. Sie kommt aufgrund der zusätzlich wirkenden Reibungskraft in der Flüssigkeit zustande. Nach Stokes gilt für die Kraft, mit der ein kugelförmiges Teilchen des Halbdurchmessers **r** mit konstanter Geschwindigkeit $v$ durch ein Medium der Zähigkeit $\eta$ bewegt wird:

$$F_s = 6 \pi \eta r v_z$$

Sie ist geschwindigkeitsabhängig und nimmt mit der Geschwindigkeit zu. Auf diese Weise ist es erklärlich, warum die Sinkgeschwindigkeit einer Kugel zunächst zunimmt, bis bei einer Grenzgeschwindigkeit die bremsende Reibungskraft, die aus Gravitation und Auftrieb resultierende Kraft kompensiert. Der Quotient aus der Kraft $F_s$ und der Geschwindigkeit $v_z$ ist der Reibungskoeffizient $f_R$, er wird ausgedrückt durch:

$$f_R = 6 * \pi * \eta * r$$

Wenn die Gestalt des/der Teilchen von der Kugelgestalt abweicht, dann darf diese Formel nicht mehr angewendet werden. Es ist zulässig, $f_R$ zu eliminieren, wenn irgendeine andere Eigenschaft bekannt ist, die von $f_R$ abhängt. Für den Diffusionskoeffizienten **D** der Teilchen in einer verdünnten Lösung gilt:

$$D_K = k T/6 \pi \eta r$$

Dabei ist es nicht unerheblich welche dynamische Viskosität das strömende Medium besitzt.

### g) Druck [p] und Kompression

$F_n$ ist der Betrag, der senkrecht zur Fläche A wirkenden Kraftkomponente $F_n \Rightarrow$

$$p = F_n / A \ (N/m^2) = 1 \ Pascal = 1 \ Pa$$

Zur Betrachtung der Druckverhältnisse (hydrostatischen Druck) in einer Flüssigkeit muß das Gewicht der Flüssigkeit/Mediums mit einbezogen werden, der Schweredruck muß hinzugefügt werden. Aufgrund der Gewichtskraft $F_n = m*g$ in einer Flüssigkeitssäule wirkt sich am Boden (Schicht) der Schweredruck aus. Das Volumen einer Flüssigkeitssäule $V = A * h$, mit der Dichte $\sigma$ ist die Masse dieser Säule $m = \sigma * A * h \Rightarrow$ der Schweredruck am Boden der Flüssigkeitssäule

$$p_s = \sigma * g * h \Rightarrow$$

Der erzeugte Schweredruck ist bei einer bestimmten Substanz mit der Dichte $\sigma$ nur der Höhe h der Flüssigkeitssäule proportional. Dies bedeutet, daß in einer beliebigen Tiefe $h_1$ innerhalb der Flüssigkeitssäule der Schweredruck $p_1 = \sigma * g * h_1$ beträgt. Bei einer nach oben geöffneten Flüssigkeitssäule wirkt zusätzlich der atmosphärische Druck $p_A$ auf die Flüssigkeitsoberfläche, dieser wird als Stempeldruck bezeichnet. Der gesamthydrostatische Druck p ist dann

$$p = p_A + \sigma * g * h$$

Ganz allgemein gilt, daß sich der Druck in einer bestimmten Höhe unterhalb Flüssigkeitsoberfläche aus Stempeldruck $p_{st}$ und Schwerdruck zusammensetzt ⇒

$$p = p_{st} + \sigma * g * h$$

Die Zentrifugalkraft $F_z$ auf ein Masseelement **m** im Abstand **r** von der Drehachse (Umlauffrequenz $f_{U/sec}$, Winkelgeschwindigkeit $\omega = 2\,\pi f$) ist nach dem II. Newtonschen Axiom definiert

$$F_z = m * r * (2\,\pi f)^2$$

Die Zentripetalkraft $F_p$, ist der von $F_z$ gleich. Diese kann aber nur durch den Flüssigkeitsdruck p aufgebracht werden, der danach an der Vorder- und Rückseite A (gesamtumgebend), bei r und r + δr des Masseelementes sich gerade so unterscheidet, daß die Kraftdifferenz gleich $F_z$ ist

$$F_p = \sigma_{fl} * A * \delta r * r * \omega \Rightarrow$$

In einer Zentrifuge wird folglich die Schwerebeschleunigung durch die Zentrifugalbeschleunigung $a_z = r * \omega$ ersetzt, und diese ist folglich nur noch vom Abstand r der Drehachse abhängig. Sie steigt proportional zu diesem Abstand an. Es ist nun möglich die Funkton $p = p_r$ durch eine einfache Integration aus der Druckgleichung darzustellen,

$$p = p_0 + \sigma_{fl} * \tfrac{1}{2} * \omega^2 (r^2 - r_0^2)$$

dabei ist $r_0$ der innere Radius, bei dem der Druck $p = p_0$ beträgt. Bei den weiteren Überlegungen muß nun berücksichtigt werden ob die entsprechende Flüssigkeit kompressibel ist. Blut als Gemisch ist ein inkompressibles Medium. Die einzelnen Komponenten wiederum besitzen ein unterschiedliches elastisches Verhalten, das Elastizitätsmodul, und sind damit komprimierbar. Ob sich die Komprimierbarkeit auf das Volumen der einzelnen Komponenten auswirkt, hängt von der Größe des Kompressionsmoduls ab.

Die auf einen Querschnitt bezogene, senkrecht zur Oberfläche eines Körpers angreifende Kraft $\sigma = F/A$ [Einheit = 1N/m2 = 1 Pascal (Pa)] heißt Normalspannung. Positive Spannungen werden als Zugspannungen, negative als Druckspannungen definiert. Nach dem Hookeschen Gesetz $\delta l = \alpha * Fl_0/A$ ($\alpha$ Materialkonstante) ist $\alpha$ der Dehnungskoeffizient, sein Kehrwert

$$1/\alpha \quad = \quad E$$

ist das Elastizitätsmodul. Dieses hat die gleiche Einheit wie die Spannung $\sigma$. Wird auf einen Körper ein allseitiger hydrostatischer Druck $-\sigma = \delta p$ ausgeübt, so ist die Volumenänderung dreimal so groß:

$$\delta V/V_0 = [3\,(1 - 2\mu)/E]\delta p = -\chi\delta p$$

dabei gilt

$$\chi = -(1/V_0)\,(\delta V/\delta p) = [3\,(1 - 2\mu)/E] = 1/K$$

Der reziproke Wert $1/\chi = K$ heißt Kompressionsmodul. Dieser ist ein Maß für die Volumenelastizität fester und flüssiger Stoffe. Da das Volumen aller Körper durch Druck verkleinert wird ($\delta V > 0$ für $\delta p > 0$), gilt stets

$$\chi > 0 \text{ und daher } \mu < \tfrac{1}{2}$$

Die Kompressibilität von Flüssigkeiten ist temperaturabhängig und übersteigt die von Festkörpern um ein bis zwei Zehnerpotenzen. So ist die von Wasser bei 293 Kelvin $4{,}591 * 10^{-10}$ Pa$^{-1}$ = $4{,}591 * 10^{-5}$ bar$^{-1}$. ⇒

$$\delta V = -\chi\,\delta p\,V_0 \Rightarrow$$

$$V_{Neu} = V_0 - (\chi \, \delta p \, V_0)$$

Wenn auf alle Flächen eines Elementes derselbe spezifische Druck ausgeübt wird, dann wird eine gleichförmige Kompression erzielt. Das Kompressionsmodul K ist das Verhältnis von Kompressionskraft pro Flächeneinheit / Volumenänderung pro Volumeneinheit. Da alle Materialien ihr Volumen verkleinern, wenn sie einem äußeren Druck ausgesetzt sind, wird ein Minuszeichen eingeführt um K positiv zu erhalten. Der von der Flüssigkeit ausgeübte Druck ist äquivalent zu seiner Schubspannung, und die relative Volumenabnahme (-δV/V) ist die Kompressionsverformung. Das Konzept von Kompressionsmodul und Kompressibilität kann sowohl auf Flüssigkeiten und Gase als auch auf Festkörper angewendet werden. Flüssigkeiten und Festkörper sind relativ inkompressibel; sie haben folglich kleine Werte für die Kompressibilität und große für das Kompressionsmodul. Nach dem Gesetz von Boyle und Mariotte (bei konstanter Temperatur) kann wie folgt als Näherung (Einstein Ann. Physik 1905) die Zustandsgleichung für ideale Gase angewendet werden.

$$p \, V = K \, n \, R \, T \Rightarrow$$

$$K = p \, V / n \, R \, T$$

[ p = Druck ; V = Volumen; n = Anzahl Mol; R = Boltzmann-Konstante; T= Temperatur (Kelvin) ]
Um ein entsprechendes $\chi$ für die einzelnen Komponenten zumindest nährungsweise zu erhalten, wird davon ausgegangen, daß die Volumenbestimmungen der Zellkomponenten bei normaler Raumtemperatur (293 Kelvin) und entsprechendem normalen Atmosphärendruck ermittelt werden. Da biologische Elemente zu weit über 90% aus Wasser bestehen, wurde das $\chi$ für Wasser mitberücksichtigt. Die Zellmembranverdichtung sowie Zellkernverdichtung bei entsprechenden Drücken wurde nicht berücksichtigt.

**h) Stromstärke**
Stromstärke (Volumenstrom)
Bei einer inkompressiblen Flüssigkeit wie Blut ist der Volumenstrom durch jede Ebene der Flüssigkeit gleich groß:

$$V_{st} = v * A = konstant = cm^3 / min$$

**i) Sedimentation**
Läßt man einen Körper der Masse *m* (z.B. eine Kugel) in einer viskösen Flüssigkeit frei fallen, dann nimmt er nach einer gewissen Anlaufbewegung eine konstante Fallbeschleunigung an, die *Sedimentationsgeschwindigkeit* genannt wird. Sie ist durch das Gleichgewicht bestimmt, das zwischen den folgenden Kräften besteht: Die Gewichtskraft $F_G = mg$, die vertikal nach unten gerichtet ist, die Auftriebskraft $F_A = m_{fl}g$ ($m_{fl}$ Masse des verdrängten Flüssigkeitsvolumens) die vertikal nach oben gerichtet ist und die Reibungskraft $F_R$ die der Geschwindigkeit des Körpers entgegengerichtet ist. An dem sinkenden Körper haftet eine dünne Flüssigkeitsschicht, und daher ist für die Reibung tatsächlich die innere Reibung der Flüssigkeit bestimmend, nicht die Reibung zwischen fallendem Körper und Flüssigkeit. Für die Reibungakraft als Funktion der Geschwindigkeit gilt, daß sie proportional und entgegengesetzt zur Geschwindigkeit gerichtet ist (negatives Vorzeichen). Daraus folgt

$$F_K = - f_R \, \vec{v}$$

Speziell, wenn der Körper eine Kugel vom Radius *r* ist, gilt die Stokes'sche Formel

$$F_K = - 6\pi \, r \, \eta \, \vec{v} \qquad f_R = 6 \, \pi \, r \, \eta$$

wobei $\eta$ die dynamische Viskosität (Plasma = $1{,}73 * 10^{-3}$ Ns m$^{-2}$) beträgt.
Konstante Sinkgeschwindigkeit $v_s$ tritt dann ein, wenn die Summe der Kräfte gleich Null ist. Aus dieser Tatsache läßt sich die Sedimentationsgeschwindigkeit berechnen:

$$v_s = \frac{m - m_{fl}}{f_R} \, g = m \, \frac{1 - \sigma_{fl}/\sigma}{f_R} = \frac{2}{9} \, r^2 \, \sigma \, \frac{1 - \sigma_{fl}/\sigma}{\eta} \, g$$

Da die Sedimentationsgeschwindigkeit von der Schwerefeldstärke abhängig ist (proportional), und diese wiederum von Erdoberflächenbeschleunigung **g** (= 9,81 m/s) ist es möglich Sinkgeschwindigkeiten von Körpern zu ermitteln, In Zentrifugen ist **g** durch die Zentrifugalbeschleunigung $a_z$ zu ersetzen. Die Sedimentationsgeschwindigkeit kann damit variiert werden. Wenn man das in einer Zentrifuge auftretende Kraftfeld betrachtet, dann wird **g** durch $\omega^2$ **$r_Z$** ersetzt; dabei ist $\omega$ die Winkelgeschwindigkeit (= $2\pi f_{U/sec}$) $r_Z$ der Abstand des betrachteten Teilchens von der Rotationsachse $\Rightarrow$

$$f_R = (dr_Z/dt) = (1 - v_z{}^*\sigma) * m * \omega^2 * r_Z$$

Wenn man nun annimmt, daß die Konstante $f_R$ im Diffusionsgesetz ($D = k\,T/6\,\pi\,\eta\,r_K$) und im Gesetz für die Sedimentation dieselbe ist, dann kann sie eliminiert werden und man erhält:

$$v_s = M * D_K * (1 - v_z{}^*\sigma)/RT$$

Da bei großen „Molekülen (Zellen)" weder über die Gestalt noch über das Reibungsgesetz genaue Angaben gemacht werden können, kann man die Möglichkeit, die Masse m eines Makromoleküls durch seine molare Masse zu ersetzen, nutzen.

$$M_{molar} = m * N_A = g/mol$$

Für den Reibungskoeffizienten $f_R$ zieht man den Zusammenhang mit dem Diffusionskoeffizienten $D_K$ heran. $D_K = k * T/f_R$ , diese

Beziehung wurde 1905 durch Einstein abgeleitet $\Rightarrow$

$$N_A * k * T = RT$$

$$v_s = M_{molar} * D_K * (1 - \sigma_{fl}/\sigma)/RT * r * \omega^2$$

Für die Sedimentationsgeschwindigkeit nach Svenberg gilt:

$$s = (\delta r/(\delta t)/ \omega^2\, r = \text{Sedimentationskonstante} \Rightarrow$$

$$v_s = c * r * \omega^2 {}^* M_{molar} * (1 - v_{sp} * \sigma)\, (1/f_R)$$

[$v_{sp}$ = partielle spezifische Molvolumen; c = Konzentration = Menge an Solvendum in der Volumeneinheit der Lösung (mol dm$^{-3}$)] [$v_{sp}$ = man gibt in das Volumen einer Lösung n Mole eines Elementes A und mißt die Volumenzunahme der Lösung bei konstanter Temperatur und konstantem Druck $\Rightarrow$

$$v_{sp} = (\delta V/\delta n_A)]$$

Um das Sedimentationsgleichgewicht für die einzelnen Komponenten zu bestimmen, im Gleichgewicht sind die Sedimentationsgeschwindigkeiten ausgeglichen kann durch Integration z.B. Abstand 1 gleich $r_1$ und Abstand 2 gleich $r_2$, das molare Massenmittel M bestimmt werden.

$$\overline{M} = [2\,RT\,\ln(c_2/c_1)] \,/\, [(1\text{-}\nu_{sp}\sigma)\,\omega^2\,(r_2{}^2 - r_1{}^2)]$$

Tabelle 3a

|  | $\nu_{sp}$ | K | $\chi$ | $f_R$ |
|---|---|---|---|---|
|  | $cm^3g^{-1}$ | $Nm^{-2}$ | $Nm^{-2}$ | $Nscm^{-1}$ |
| Thrombo | 0,9615 | 1,9421E+09 | 5,14904E-10 | 4,48E-06 |
| Lympho | 0,9470 | 1,9127E+09 | 5,22826E-10 | 1,39E-05 |
| CD34+ | 0,9443 | 1,9073E+09 | 5,24311E-10 | 1,79E-05 |
| Mono | 0,9407 | 1,9001E+09 | 5,26291E-10 | 2,20E-05 |
| PNC | 0,9200 | 1,8581E+09 | 5,38174E-10 | 1,71E-05 |
| Ery | 0,9132 | 1,8446E+09 | 5,42135E-10 | 1,22E-05 |

Tabelle 3b

|  | $f_R$ | $D_K$ | $D_K$ | RT |
|---|---|---|---|---|
|  | $Nsm^{-1}$ | $cm^2s^{-1}$ | $m^2s^{-1}$ | J/mol |
| Thrombo | 4,48E-08 | 9,11E-11 | 9,11E-14 | 2,4611E+03 |
| Lympho | 1,39E-07 | 2,95E-11 | 2,95E-14 | 2,4611E+03 |
| CD34+ | 1,79E-07 | 2,28E-11 | 2,28E-14 | 2,4611E+03 |
| Mono | 2,20E-07 | 1,86E-11 | 1,86E-14 | 2,4611E+03 |
| PNC | 1,71E-07 | 2,39E-11 | 2,39E-14 | 2,4611E+03 |
| Ery | 1,22E-07 | 3,34E-11 | 2,34E-14 | 2,4611E+03 |

Damit ist in einem Gemisch aus Stoffen unterschiedlicher molarer Massen die Sedimentationsgeschwindigkeit verschieden. Es wird folglich eine Entmischung des Gemisches eintreten, die sich als Dichteänderung der Flüssigkeit bemerkbar macht.

Tabelle 4a

|  | Volumen | r | d | Dichte |
|---|---|---|---|---|
|  | Liter | m | m | $kg/dcm^3$ |
|  |  |  |  |  |
| Vollblut |  |  |  | 1,060 |
| Plasma |  |  |  | 1,030 |
| Thrombo | 1,20E-14 | 1,38E-06 | 2,75E-06 | 1,040 |
| Lympho | 7,20E-14 | 2,43E-05 | 4,86E-06 | 1,056 |

Tabelle 4a (fortgesetzt)

|  | Volumen | r | d | Dichte |
|---|---|---|---|---|
|  | Liter | m | m | kg/dcm$^3$ |
| CD34+ | 1,00E-13 | 2,83E-05 | 5,66E-05 | 1,059 |
| Mono | 1,33E-13 | 3,14E-05 | 6,29E-05 | 1,063 |
| PNC | 2,40E-13 | 3,86E-05 | 7,71E-05 | 1,087 |
| Ery | 9,20E-14 | 2,80E-06 | 5,60E-06 | 1,095 |

Tabelle 4b

|  | Dichte | Dichte | Masse | $M_{molar}$ |
|---|---|---|---|---|
|  | g/ml | g/fl | kg/Zelle | kg/mol |
|  |  |  |  |  |
| Vollblut | 1,060 | 1,060E-15 |  |  |
| Plasma | 1,030 | 1,030E-15 |  |  |
| Thrombo | 1,040 | 1,040E-15 | 1,25E-14 | 7,52E+07 |
| Lympho | 1,056 | 1,056E-15 | 7,60E-14 | 4,58E+08 |
| CD34+ | 1,059 | 1,059E-15 | 1,06E-13 | 6,38E+08 |
| Mono | 1,063 | 1,063E-15 | 1,42E-13 | 8,53E+08 |
| PNC | 1,087 | 1,087E-15 | 2,72E-13 | 1,64E+09 |
| Ery | 1,095 | 1,095E-15 | 1,01E-13 | 6,07E+08 |

Grundsätzlich erhält man aber keine völlig scharfe Trennung der Komponenten, sondern für jede Komponente kommt es zu einer Verteilung der Anzahldichte, die stets ihr Maximum beim größtmöglichen Abstand von der Drehachse hat. Von diesem Maximum klingt die Dichte nach innen ab und verläuft nach der gleichen Gesetzmäßigkeit wie die Dichte der atmosphärischen Luft.

$$P = p_0 \exp [ - (M_{molar} \cdot g/RT) \cdot h]$$

Ersetzt man nun die in der o.g. Formel **g** durch $\mathbf{a_z}$ so erhält man die Anzahldichte der Komponente mit der molaren Masse $M_{molar}$

$$n_r = n_0 \exp \left( [M_{molar} / 2\,RT] \cdot (1 - \sigma_{fl}/\sigma) \cdot (r^2_{max} - r^2) \cdot \omega^2 \right)$$

**Optimierung der maschinellen Funktionsparametereinstellung von Apheresemaschinen**

**1. Zentrifugenumdrehungsgeschwindigkeit (rpm)**
(Anhang Seite 41; Graphik 1 und 2)

Um eine optimale Sammlung von spezifischen Zellkomponenten zu erhalten, wurden die o.g. physikalischen und biophysikalischen Erkenntnisse herangezogen. Dabei ist es notwendig die Anzahldichte/$\mu$l bzw. /ml innerhalb der Zentrifugen so zu berücksichtigen, daß ein optimales Verhältnis der zu sammelnden Zellkomponenten zu den nicht zu sammelnden Zellkomponenten vorliegt, hierbei müssen insbesondere die Sedimentationsgeschwindigkeiten der einzelnen Komponenten, sowie die Zeit, der sie der Zentrifugalkraft ausgesetzt sind berücksichtigt werden. Allgemein kann für die Zentrifugalwirkung formuliert werden,

$$G = r * [Umin^{-1}/1000]^2 * 11,18 \ m \ s^{-2} \Rightarrow$$

$$a_r = r * \omega^2 = r * (2 \pi f)^2 \Rightarrow$$

$$a_z = m * r * \omega^2 = m * r * (2 \pi f)^2$$

Für die Zentrifugalwirkung einer Zentrifuge, Abstand von der Zentrifugenachse konstant, stehen die g-Zahl und Zentrifugationsdauer in reziprokem Verhältnis, sodaß bei Verdopplung der Zentrifugendauer die g-Zahl halbiert werden kann und umgekehrt. Der Faktor 11,18 m s$^{-2}$ leitet sich von der Erdbeschleunigung ab. Um diese Optimierung zu erreichen, müssen die o.g. Kräfte, Geschwindigkeiten welche innerhalb der Zentrifuge herrschen, möglichst genau bestimmt werden, woraus sich der Trennungsoptimierungskoeffizient = $T_{OK}$ oder auch Separationsfaktor SF für die jeweiligen zu sammelnden Zellkomponenten ermitteln läßt.

$$T_{OK} = [r * (2 \pi f)^2 * t * Fv_K * 1/t]/ [GF/g]$$

1.1. Die Sammlung von Blutstammzellen bzw. mononukleären Zellen = MNZ ist dadurch gekennzeichnet, daß deren Intervall für $T_{OK(MNZ)}$ = SF zwischen [140 — 190] liegt, genau berechnet bei

$$T_{OK(MNZ)} = SF = 176$$

Gegenwärtig werden für die Sammlung dieser Zellkomponenten Mindest-werte gleich $T_{OK(MNZ)}$ = SF von [400 —500] 500 empfohlen.

1.2. Die Sammlung von Thrombozyten, bei Blutflußgeschwindigkeiten zwischen 30 und 100 ml/min, ist dadurch gekennzeichnet, daß das Intervall für $T_{OK(MNZ)}$ = SF zwischen [600 — 700] liegt, genau berechnet bei

$$T_{OK(Tk)} = SF = 649$$

Gegenwärtig werden für die Sammlung dieser Zellkomponente Mindest-werte gleich $T_{OK(MNZ)}$ = SF von [1000 —3700] verwendet.

Da der $T_{OK}$ = SF eine Konstante ist, ist die Umdrehungsfrequenz (rpm) der Zentrifuge vom Gesamteinlaß (GF$_{ml/min}$), Volumen input (ml/min) in die jeweilige Zentrifugenkammer abhängig und durch die Formel 1 gekennzeichnet $\Rightarrow$

## Formel 1

$$rpm = a * GF_{ml/min}{}^{b1}$$

(a und b$_1$ sind Konstanten, reelle numerische Werte, die bei der Gewinnung der definierten Zellkomponenten verschieden sind)

**2. Thrombozytenreicher Plasmavolumenanteil ml/min (TrPVat $_{ml/min}$)**

(Anhang Seite 42; Graphik 3)

Für die Ermittlung der optimalen Stelle, an der sich die gewünschten zu sammelnden Zellkomponenten in einem geschlossenen Zentrifugenkanal befinden, kommen die Gesetzmäßigkeiten der Sedimentation zur Anwendung. Es werden sich die Zellkomponenten mit der größten Dichte (spez. Gewicht = kg/dcm3) am maximalen, während die mit der geringsten Dichte am minimalsten Abstand von der Drehachse anreichern. Um die o.g. Stelle in dem getrennten Gemisch zu ermitteln, muß beachtet werden, daß Eine, wenn auch nicht völlig scharfe Trennung der einzelnen Komponenten des Gemisches, aber doch eine weitgehende Entmischung eingetreten ist und sich somit eine Dichteänderung des „Gemisches" eingestellt hat. Um die determinierte Zellkomponente aus dem neu entstandenen „Gemisch" sammeln zu können, muß der Volumenanteil TrPVat$_{ml/min}$ (z.B. thrombozytenreicher Plasmaanteil) des „Gemisches", welcher sich über der determinierten Zellkomponente gebildet hat entfernt werden. Dieser Vorgang kann z.B. durch eine kontinuierliche Pumpe durchgeführt werden. Zur Gewinnung von thrombozytenreichen Plasma ist dies nicht notwendig, da das thrombozytenreiche Plasma aufgrund der geringsten Dichte die oberste Schicht in dem neu entstandenen „Gemisch" bildet. Die Ermittlung der Pumpengeschwindigkeit des TrPVat$_{ml/min}$ wird durch folgende Formel gekennzeichnet:

## Formel 2

$$TrPVat_{ml/min} = a_{TrPVat} * e^{-b * Korr.-Hkt.}$$

Hkt. = Hämatokrit des Vollblutes = der Volumen - Prozentanteil, den in 1 µl Vollblut die Erythrozyten einnehmen. Hkt. (%) * 10 = MCV ($\mu m^3$) * Ery $10^6$/µl

Näherungswerte des Volumenanteiles (%) der einzelnen spezifischen Zellkomponenten (Zk spef.) in 1 µl Vollblut =

1. Für Thrombozyten (%) * $10^6$ = Anzahldichte/µl (Azd$_{\mu l}$) * Zellvolumen fl (ZV$_{fl}$) z.B. Azd$_{\mu l}$ = 166 * $10^3$; ZV$_{fl}$ = 1,2 * $10^{-14}$ dcm$^3$ $\Rightarrow$ 0,199 %

2. Für die Leukozyten, muß durch die Differentialblutbestimmung bzw. mittels Durchflußzytometrie die relativen Prozentanteile innerhalb der Leukozyten ermittelt werden: z.B. (Leukozyten =) Azd$_{\mu l}$ = 35.6 * $10^3$ $\Rightarrow$

2.1. Für Lymphozyten (5%) Azd$_{\mu l}$ = 1.78 * $10^3$; ZV$_{fl}$ = 7,2 * $10^{-14}$ dcm$^3$ $\Rightarrow$ 0.013 %

2.2. Für CD34+ (1%) Azd$_{\mu l}$ = 3,56 * $10^2$; ZV$_{fl}$ = 10 * $10^{-14}$ dcm$^3$ $\Rightarrow$ 0.004 %

2.3. Für Monozyten (8%) Azd$_{\mu l}$ = 2,85 * $10^3$; ZV$_{fl}$ = 13 * $10^{-14}$ dcm$^3$ $\Rightarrow$ 0.038 %

2.3. Für PNC (86%) Azd$_{\mu l}$ = 30,6 * $10^3$; ZV$_{fl}$ =25 * $10^{-14}$ dcm$^3$ $\Rightarrow$ 0.765 %

3. Für Erythrozyten Azd$_{\mu l}$ = 4,79 * $10^6$; ZV$_{fl}$ = 9,4 * $10^{-14}$ dcm$^3$ $\Rightarrow$ 45,02 %

Daraus ergibt sich ein „zellfreier" Plasmaanteil von 54%.

Aus den oben gezeigten Daten wird ersichtlich, daß der Hämatokrit, gemessen am Gesamtzellvolumenanteil etwa 1 % höher liegt $\Rightarrow$ 46%.

Die nachfolgenden Tabelle 5a und 5b kennzeichnen durch die Anzahldichteformel wie sich die Anzahldichte der Zellkomponenten auf der Grundlage von

$$n_r = n_0 \exp ( [M_{molar}/ 2 RT] * (1 - \sigma_{fl}/\sigma) * (r^2_{max} - r^2) * \omega^2 )$$

unter definierter Krafteinwirkung verändert.

Als Ausgangspunkt wurden die Daten des Beispieles der Beschreibung für TrPVat und der Umdrehungsgeschwindigkeiten (Graphik 1) eingesetzt.

Tabelle 5a

| GF | Thrombo | Lympho | CD34+ |
|----|---------|--------|-------|
| 30 | 2,92E+06 | 3,62E+04 | 7,40E+03 |
| 40 | 2,97E+06 | 3,68E+04 | 7,51E+03 |
| 50 | 3,01E+06 | 3,72E+04 | 7,59E+03 |

Tabelle 5a (fortgesetzt)

| GF | Thrombo | Lympho | CD34+ |
|---|---|---|---|
| 60 | 3,04E+06 | 3,75E+04 | 7,65E+03 |
| 70 | 3,07E+06 | 3,78E+04 | 7,71E+03 |
| 80 | 3,09E+06 | 3,80E+04 | 7,75E+03 |
| 90 | 3,11E+06 | 3,82E+04 | 7,80E+03 |
| 100 | 3,12E+06 | 3,84E+04 | 7,83E+03 |
| 110 | 3,14E+06 | 3,86E+04 | 7,87E+03 |
| 120 | 3,15E+06 | 3,87E+04 | 7,90E+03 |
| 130 | 3,17E+06 | 3,89E+04 | 7,93E+03 |
| 140 | 3,18E+06 | 3,90E+04 | 7,95E+03 |
| 150 | 3,19E+06 | 3,91E+04 | 7,98E+03 |

Tabelle 5b

| GF | Mono | PNC | Ery |
|---|---|---|---|
| 30 | 6,04E+04 | 6,86E+05 | 1,03E+08 |
| 40 | 6,12E+04 | 6,94E+05 | 1,04E+08 |
| 50 | 6,19E+04 | 7,01E+05 | 1,06E+08 |
| 60 | 6,24E+04 | 7,07E+05 | 1,06E+08 |
| 70 | 6,28E+04 | 7,12E+05 | 1,07E+08 |
| 80 | 6,32E+04 | 7,16E+05 | 1,08E+08 |
| 90 | 6,36E+04 | 7,19E+05 | 1,08E+08 |
| 100 | 6,39E+04 | 7,22E+05 | 1,09E+08 |
| 110 | 6,41E+04 | 7,25E+05 | 1,09E+08 |
| 120 | 6,44E+04 | 7,28E+05 | 1,10E+08 |
| 130 | 6,46E+04 | 7,30E+05 | 1,10E+08 |
| 140 | 6,48E+04 | 7,33E+05 | 1,10E+08 |
| 150 | 6,50E+04 | 7,35E+05 | 1,11E+08 |

Aus den oben gezeigten Anzahldichteveränderungen ist ersichtlich, daß die zellulären Komponenten, auch wenn sie keinen großen prozentualen Volumenanteil annehmen, im Gesamt — Komplex beachtet werden müssen, da die jeweils determinierte Zellkomponente nach der Entfernung des Plasmas ihren Volumendichte-Anteil verändert. Aufgrund dieser Gegebenheit wurde für die Ermittlung von $TrPVat_{ml/min}$ der Hämatokrit-Korrekturfaktor eingeführt.

In den nachfolgenden Tabelle 6 sind zur Vereinfachung der prozentuale Korrekturfaktor für den Hämatokrit in Abhängigkeit vom PNC-Anteil = Korr.-Hkt. dargestellt.

Tabelle 6

| Leukozytenwerte pro/Mikroliter Range | PNC — Anteil | PNC — Anteil | Abweichung Hämatokrit (Labor) | Abweichung Hämatokrit n. Berechnung | Hämtokrit für TrP-Vat |
|---|---|---|---|---|---|
| bis 5000 | | | | | |

Tabelle 6 (fortgesetzt)

| Leukozytenwerte pro/Mikroliter Range | PNC — Anteil | PNC — Anteil | Abweichung Hämatokrit (Labor) | Abweichung Hämatokrit n. Berechnung | Hämtokrit für TrP-Vat |
|---|---|---|---|---|---|
| | PNC < 2500 | | 3% | -0,75% | [(Hkt*103)*99,25] |
| | | 2500 > PNC | -3% | 5% | [(Hkt*97)*105] |
| 5000 — 10000 | | | | | |
| | PNC < 6000 | | 3% | -5,50% | [(Hkt*103)*94,5] |
| | | PNC < 9000 | -3% | 7,5% | [(Hkt*97)*107,5] |
| 10000 — 15000 | | | | | |
| | PNC<10000 | | 3% | -5,5% | [(Hkt*103)*94,5] |
| | | PNC < 13000 | -3% | 7,5% | [(Hkt*97)*107,5] |
| 15000 — 20000 | | | | | |
| | PNC<15000 | | 3% | -5,5% | [(Hkt*103)*94,5] |
| | | PNC < 19000 | -3% | 7,5% | [(Hkt*97)*107,5] |
| 20000 — 25000 | | | | | |
| | PNC<19000 | | 3% | -5,50% | [(Hkt*103)*94,5] |
| | | PNC < 21000 | -3% | 7,5% | [(Hkt*97)*107,5] |
| 25000 — 30000 | | | | | |
| | PNC<24000 | | 3% | -5,50% | [(Hkt*103)*94,5] |
| | | PNC < 26000 | -3% | 7,5% | [(Hkt*97)*107,5] |
| 30000 — 35000 | | | | | |
| | PNC<27000 | | 3% | -5,5% | [(Hkt*103)*94,5] |
| | | PNC<29000 | -3% | 7,5% | [(Hkt*97)*107,5] |
| 35000 — 40000 | | | | | |
| | PNC<33000 | | 3% | -5,50% | [(Hkt*103)*94,5] |
| | | PNC<37000 | -3% | 7,5% | [(Hkt*97)*107,5] |
| 40000 — 45000 | | | | | |
| | PNC<38000 | | 3% | -5,50% | [(Hkt*103)*94,5] |
| | | PNC<42000 | -3% | 7,5% | [(Hkt*97)*107,5] |
| 45000 — 55000 | | | | | |
| | PNC<40000 | | 3% | -5,50% | [(Hkt*103)*94,5] |
| | | PNC>43000 | -3% | 7,5% | [(Hkt*97)*107,5] |

Zur Berechnung des Exponentenfaktors „ $b$ „ von $TrPVat_{ml/min}$ ist $GF_{ml/min}$ nötig.

$$b_{TrPVat} = m_1 : e^{\,b1 \,*\, GF\,(ml/min)}$$

($m_1$ und $b_1$ sind konstante Faktoren, die bei der Gewinnung der definiertern Zellkomponenten verschieden sind)

Zur Berechnung der Konstanten „ $a$ „ von $TrPVat_{ml/min}$ ist $GF_{ml/min}$ nötig.

$$a_{TrPVat} = m_2 : e^{\,-\,b2 \,*\, GF(ml/min)}$$

($m_2$ und $b_2$ sind konstante Faktoren, die bei der Gewinnung der definierten Zellkomponenten verschieden sind)

**3. Sammelvolumenflußberechnung = S$_{VF}$ (ml/min)**
(Anhang Seite 43 ; Graphik 4)

Zur Berechnung des Sammelflusses S$_{VF}$ der verschiedenen Zellkompo-nenten wurde die Volumenstromgeschwindigkeit, die Zellkomponenten-volumenänderung, die veränderte Anzahldichte und TrPVat$_{ml/min}$, welche durch die Zentrifugalkraft bedingt ist, berücksichtigt. S$_{VF}$ wird durch die Formel 3 gekennzeichnet

**Formel 3**

$$S_{VF} \text{ (ml/min)} = [(\ln(\text{Korr.Hkt.}) * A_1) - (A_2 * e^{-b_3 * \text{Korr.Hkt.}})] \pm F$$

$$A_1 = m_1 * e^{(bSVF1) * x} \quad (x = GF_{ml/min})$$

(m$_1$ und bS$_{VF1}$ sind konstante Faktoren, die bei der Gewinnung der definiertem Zellkomponenten verschieden sind)

$$A_2 = m_2 * e^{(bSVF2) * x} \quad (x = GF_{ml/min})$$

(m$_2$ und bS$_{VF2}$ sind konstante Faktoren, die bei der Gewinnung der definierten Zellkomponenten verschieden sind)

$$b_3 = m_3 * e^{(bSVF3) * x} \quad (x = GF_{ml/min})$$

(m$_3$ und bS$_{VF3}$ sind konstante Faktoren, die bei der Gewinnung der definierten Zellkomponenten verschieden sind)

F = Korrekturfaktor

Für Gesamteinlaßflüsse < 123 ml/min bzw. < 51 ml/min beträgt er — **0,2;**
für Gesamteinlaßflüsse 123 — 51 ml/min **+ 0,2**
Volumenveränderung von Zellkomponenten bei bestimmten Volumenein-laßgeschwindigkeiten (ml/min), auf der Grundlage definierter Winkelgeschwindigkeiten (az A1 und az A4) bei konstantem Trennungsoptimierungsfaktor gleich Separationsfaktor.

Tabelle 7a

| GF | p | | Thrombo | Lympho | CD 34+ |
|---|---|---|---|---|---|
| ml/min | N/m$^2$ | bar | dcm$^3$ | dcm$^3$ | dcm$^3$ |
| | 1,01E+05 | 0,9807 | 1,2000E-14 | 7,2000E-14 | 1,0000E-13 |
| 21 | 1,98E+05 | 1,9756 | 1,1999E-14 | 7,1997E-14 | 9,9995E-14 |
| 31 | 2,97E+05 | 2,9659 | 1,1999E-14 | 7,1993E-14 | 9,9990E-14 |
| 41 | 3,95E+05 | 3,9546 | 1,1998E-14 | 7,1990E-14 | 9,9986E-14 |
| 51 | 4,95E+05 | 4,9469 | 1,1998E-14 | 7,1986E-14 | 9,9981E-14 |
| 62 | 5,94E+05 | 5,9367 | 1,1997E-14 | 7,1983E-14 | 9,9976E-14 |
| 72 | 6,94E+05 | 6,9351 | 1,1997E-14 | 7,1979E-14 | 9,9971E-14 |

Tabelle 7a (fortgesetzt)

| GF | p | | Thrombo | Lympho | CD 34+ |
|---|---|---|---|---|---|
| ml/min | $N/m^2$ | bar | $dcm^3$ | $dcm^3$ | $dcm^3$ |
| 82 | 7,92E+05 | 7,9239 | 1,1996E-14 | 7,1976E-14 | 9,9966E-14 |
| 92 | 8,91E+05 | 8,9093 | 1,1995E-14 | 7,1972E-14 | 9,9961E-14 |
| 103 | 9,90E+05 | 9,9038 | 1,1995E-14 | 7,1969E-14 | 9,9957E-14 |
| 113 | 1,09E+06 | 10,8998 | 1,1994E-14 | 7,1965E-14 | 9,9952E-14 |
| 123 | 1,19E+06 | 11,8902 | 1,1994E-14 | 7,1962E-14 | 9,9947E-14 |
| 133 | 1,29E+06 | 12,8682 | 1,1993E-14 | 7,1959E-14 | 9,9942E-14 |
| 144 | 1,39E+06 | 13,8561 | 1,1993E-14 | 7,1955E-14 | 9,9937E-14 |
| 154 | 1,49E+06 | 14,8507 | 1,1992E-14 | 7,1952E-14 | 9,9933E-14 |

Tabelle 7a

| GF | p | | Mono | PNC | Ery |
|---|---|---|---|---|---|
| ml/min | $N/m^2$ | bar | $dcm^3$ | $dcm^3$ | $dcm^3$ |
| | 1,01E+05 | 0,9807 | 1,3300E-13 | 2,4000E-13 | 9,2000E-14 |
| 21 | 1,98E+05 | 1,9756 | 1,3299E-13 | 2,3999E-13 | 9,1995E-14 |
| 31 | 2,97E+05 | 2,9659 | 1,3299E-13 | 2,3998E-13 | 9,1991E-14 |
| 41 | 3,95E+05 | 3,9546 | 1,3298E-13 | 2,3996E-13 | 9,1986E-14 |
| 51 | 4,95E+05 | 4,9469 | 1,3297E-13 | 2,3995E-13 | 9,1982E-14 |
| 62 | 5,94E+05 | 5,9367 | 1,3297E-13 | 2,3994E-13 | 9,1977E-14 |
| 72 | 6,94E+05 | 6,9351 | 1,3296E-13 | 2,3993E-13 | 9,1972E-14 |
| 82 | 7,92E+05 | 7,9239 | 1,3295E-13 | 2,3992E-13 | 9,1968E-14 |
| 92 | 8,91E+05 | 8,9093 | 1,3295E-13 | 2,3991E-13 | 9,1963E-14 |
| 103 | 9,90E+05 | 9,9038 | 1,3294E-13 | 2,3989E-13 | 9,1959E-14 |
| 113 | 1,09E+06 | 10,8998 | 1,3294E-13 | 2,3988E-13 | 9,1954E-14 |
| 123 | 1,19E+06 | 11,8902 | 1,3293E-13 | 2,3987E-13 | 9,1950E-14 |
| 133 | 1,29E+06 | 12,8682 | 1,3292E-13 | 2,3986E-13 | 9,1945E-14 |
| 144 | 1,39E+06 | 13,8561 | 1,3292E-13 | 2,3985E-13 | 9,1940E-14 |
| 154 | 1,49E+06 | 14,8507 | 1,3291E-13 | 2,3983E-13 | 9,1936E-14 |

**4. ACD — Maschinen — Geschwindigkeit ml/min**
ist gekennzeichnet durch

Formel 4

$$\text{ACD ml/h} = M = BF * 2{,}5\ \%$$

$$\Rightarrow BF : ACD = 40 : 1$$

Dadurch erhält der Spender deutlich weniger Antikoagulanz und somit treten weniger Nebenwirkungen auf.

**5. ACD - Perfusor- Geschwindigkeit ml/h**
ist gekennzeichnet durch

Formel 5

$$\text{Perfusorgeschwindigkeit ml/h} = P = S_{VF} * 250\ \%$$

Dadurch wird gewährleistet, daß in dem gesammelten statischen Präparat gezielt ausreichend Antikoagulanz vorhanden ist.

**6. ACD - Konzentration (K) im Präparat**
ist gekennzeichnet durch

Formel 6

$$K : ACD = (S_{VF}\ \text{ml}* 250\ \% + S_{VF}\ \text{ml} * 2{,}5\ \%)$$

$$\Rightarrow K : ACD = 15 : 1$$

**Erläuterung zur Verfahrenstechnik - Verbesserung bei Veränder-ungen der spez. Proteinkonzentrationen im Blutplasma**

Werden die zellulären Elemente des Blutes durch Zentrifugation abgetrennt, so erhält man eine proteinhaltige Flüssigkeit, das **Blutplasma**. Der gewonnene Überstand des geronnenen Blutes ist das **Blutserum**.

Blutplasma ist ein komplexes Gemisch aus Glyko- und Lipoproteinen. Der Gesamtproteingehalt des Plasmas beträgt 6 — 8 g/100ml. Die nachfolgende Tabelle zeigt eine Auswahl über die Molekulargewichte bzw. der Konzentrationen im Plasma/Serum, welche den größten Anteil einnehmen.

Tabelle 8

| Element | Molekulargewicht | Konzentration | Svedberg | Dichte | d |
|---|---|---|---|---|---|
| | $10^{-3}$ | $g/dcm^3$ | $10^{-13}s$ | | nm |
| IgG | 143-149 | 9-15 | 7 S | | |
| IgA | 158-162 | 1,4-2,6 | 7 S | | |
| IgM | 800-950 | 0,7-1,8 | 19 S | | |

Tabelle 8 (fortgesetzt)

| Element | Molekulargewicht | Konzentration | Svedberg | Dichte | d |
|---|---|---|---|---|---|
| | $10^{-3}$ | $g/dcm^3$ | $10^{-13}s$ | | nm |
| $\alpha_2$-Makroglobulin | 820 | 2,6 | | | |
| Fibrinogen | 341 | 2-3 | | | |
| Haptoglobin | 100 | 1,7 | | | |
| $\beta$-Lipoproteine (LDL) | 3200 | 0,34 | | 1,000 -1,063 | 15-25 |
| Lipoproteine (VDL) | 5000 | 0,8 | | 0,94 -1,006 | 30-70 |
| $\alpha$-Lipoproteine (HDL) | 200 | 3 | | 1,063 -1,210 | 7-10 |

Erkrankungen, die eine Erhöhung der Akute-Phase-Proteine verursachen und/oder mit einer monoklonalen/polyklonalen Immunglobulinver-mehrung bzw. Immunkomplexvermehrung einhergehen, verändern:

1. die dynamische Viskosität $\eta$ des Mediums, Plasma/Serums
2. sie können sich je nach Größe des Moleküls, der Sedimentationsgeschwindigkeit und Konzentrationserhöhung (g/l) zwischen die zu sammelnden Zellkomponenten schieben und damit eine Proteinschicht bilden, welche eine Sammlung von unerwünschten Zellkomponenten z.B. bei der Stammzellsammlung (CD34+) die „Kontamination" mit polymorphkernigen Zellen deutlich erhöht.

**7. Verfahrensverbesserung**

Um annähernd normale physiologische Plasmaproteinkonzentrationen zu erhalten, welche zur optimalen Gewinnung spez. Zellkomponenten Voraussetzung sind, müssen die o.g. Proteinkonzentrationserhöhungen eliminiert werden. Dies wird erreicht, indem das Blut des Spenders einem konventionellen Plasmaaustauch (TPE = Therapeutic Plasma Exchange) unterzogen wird. Durch den TPE wird das Plasma von den zellulären Bestandteilen mittels der Zentrifugenapheresemaschine, insbesondere von den Plasmaproteinen getrennt, welche verworfen und kontinuierlich durch eine humane Proteinlösung isovolumetrisch ersetzt werden. Das „gereinigte" Plasma wird dann mit den zellulären Komponenten gemischt, dem Spender reinfundiert oder dem Zellaphereseverfahren unterzogen und dann dem Spender reinfundiert. Hierzu sind zwei unterschiedliche Verfahrensweisen möglich:

1. Das sequentielle Verfahren
oder
2. das kombinierte Verfahren
Beide Verfahren sind im Anhang auf Seite 44 durch Fluß - Diagramme gekennzeichnet.

**8. Technische Veränderungen bzw. Verfahrenstechniken**

Erläuterungen zu Skizze 1 (Anhang Seite 45)
Skizze 1 kennzeichnet die gesamten Veränderungen bzw. Verfahrenstechniken im Überblick
Bevor die entsprechenden Verfahren gestartet werden können, müssen außer den üblichen Hard-Software-Kontrollen, die Aphereseschlauchsysteme entlüftet werden. Dieser Füllvorgang geschieht gegenwärtig durch eine physiologische Kochsalzlösung = **B 1** (Na Cl 0,9%). Über einen an B 1 konnektierten Schlauch, welcher an den Entnahmeschlauch (Inlet-Line) zur Maschine führend, angeschweißt ist, wird über die Pumpe **P 1** die Lösung in das eingelegte Schlauchsystem infundiert. Am Rückführungsschlauch (Outlet-Line) von der Maschine kommend, ist ein an B 1 konnektierbarer Entlüftungsschlauch angeschweißt. Inlet-Outlet-Line bilden somit einen geschlossenen Kreislauf. Die zu entfernende Luft des Schlauchsystems wird in den Beutel **B 4** gepumpt, dadurch wird eine Kontamination der Flüssigkeit mit Luftpartikeln weitgehend ausgeschlossen. Nach Abschluß des Füll- und Spülvorganges wird sowohl Inlet- und Outlet- Line mit einer Rollklemme verschlossen.
Während des Aphereseverfahrens wird das entsprechende Blutgemisch über die Inlet-Line und mittels P 1 zur Maschine geführt. Vor Eintritt in die Zentrifugenkammer/Zentrifuge wird dem „Gemisch" über P 2 aus B3 in der Mischkammer (= MK1) Kammer in einem definierten Verhältnis das Antikoagulanz (ACD) [100 ml ACD enthalten 2,2 g wasserhaltiges Natriumzitrat, 730 mg wasserfreie Zitronensäure und 2,45 g wasserhaltige Glukose] in der Regel im Verhältnis Blutfluß:ACD von 1:10-15, zur Verhinderung der Koagulation des Gemisches zugeführt.

9. Technische Neuerung - Veränderung 1 (Skizze 1 Seite 45)
Die technische Veränderung 1 ist durch das Anschweißen eines an den Behälter **B 2** konnektierbaren Schlauches (Detailskizze Anhang Seite 47 u. 48) über eine „Y-Verbindung" (inlet-line), durch den zusätzlichen Beutel B 2 und dessen Inhalt gekennzeichnet. In dem Behältnis B 2 befindet sich eine 5%-ige Humanalbumin-Lösung. Mit dieser Lösung wird das Apheresesystem **primär** gespült, und wie oben beschrieben entlüftet. Nach einer Einwirkzeit von 5 - 10 Minuten des Humanalbumins folgt dem primären Schritt ein weiterer wie oben beschriebener Spülvorgang mit physiologischer Kochsatzlösung (NaCl 0,9%) um den größten Teil der primären Lösung wieder zu entfernen.

Bei der Primärspülung sind die Ventile der inlet-outline-line (Rasterklemme) unterhalb von B 1 geschlossen, die von B 2 (Rollklemme und Rasterklemme) geöffnet. Bei der Sekundärspülung ist das Ventil der inlet-line unterhalb von B 1 geöffnet, das von B 2 geschlossen. Das outlet-Ventil (Rasterklemme) von B 2 ist während der sekundären Spülung, solange geöffnet wie bei der Primärspülung Volumen verwendet wurde. Das von B 1 ist für das gleiche Zeitintervall geschlossen, volumen-gesteuerte Schließung und Öffnung. Nach Erreichen des Volumens wird ein akustisches Signal ausgelöst, welches den Operator zur Öffnung und Schließung der genannten Ventile auffordert. Damit wird weitgehend das Humanalbumin zurück nach B 2 befördert

Der Sinn der primären Spülung mit 5%-iger Humanalbumin-Lösung ist es die bekannten Adherierungskräfte welche Plastikoberflächen auf zelluläre Bestandteile ausüben, wenn diese mit ihnen in Kontakt treten, zu minimieren sowie deren Aggregation weitgehend zu verhindern. Zur Minimierung dieser Interaktionen, sind Humanalbumin-Moleküle sehr gut geeignet, coating von Plastikschlauchsystemen. Durch diese Primär-spülung/Entlüftung wird der Verlust von zellulären Bestandteilen bei den Zellaphereseverfahren, welche durch „Plastik" - Schlauchsysteme induziert werden, deutlich reduziert.

10. Technische Neuerung - Veränderung 2 (Skizze 1 Seite 45)

Die technische Veränderung 2 ist durch das Anschweißen eines an **P 5** konnektierbaren Schlauches, über eine „Y-Schlauchverbindung", in den Sammelschlauch, der über **P 4** zu **B 5** führt, gekennzeichnet (Detailskizze Anhang Seite 48 u. 49).

Der direkte Weg aus der Zentrifugenkammer über **P 4** nach **B 5** ist die Sammellinie für die definierten Zellkomponenten. Unterhalb von der „Y-Verbindung" von B 5 und P 5 befindet sich ein Ventil, das während des Sammelvorganges geöffnet ist, während das Ventil oberhalb von P 4 geschlossen ist und umgekehrt. Über den von P 5 kommenden Schlauch wird während des Sammelvorganges eine definierte Menge ACD pro Zeiteinheit, welche durch das Sammelvolumen pro Zeiteinheit (ml/min) definiert ist, dem Sammelvolumen direkt zugemischt. Hierdurch wird zum Einen eine gezielte Antikoagulation (volumengerecht z.B $K$ : ACD = 12 - 15: 1) für das Produkt erreicht und zum Anderen die Menge des Antikoagulanz, welche z. B. ein potentieller Spender über die Linie B3 → P 2 erhält, deutlichst verringert. Dies hat zur Folge, daß die Nebenwirkungen, welche von dem Antikoagulanz (ACD) bekannt sind auf ein Minimum reduziert werden, ohne daß das gewonnene Produkt, welches sich in einem statischen Gleichgewicht befindet, koaguliert.

11. Technische Neuerung - Veränderung 3 (Skizze 1 Seite 45)

Die technische Veränderung 3 ist durch den Einbau einer zusätzlichen Pumpe bzw. eines Perfusors, wie in der Skizze 1 dargestellt, gekenn-zeichnet. Diese Pumpe wird über ein Relais aktiviert, welches eine Verbindung zum Ventil unterhalb der „Y-Schlauchverbindung" von B 5 und P 5 besitzt. Bei Öffnung des Ventils wird ein akustisches Signal ausgelöst, dieses fordert den Operator auf die Pumpe P 5 zu aktivieren. Durch das vorher definierte Volumen/Minute, wird über den Schlauch der von P 5 läuft Antikoagulanz, berechnet nach Sammelvolumen welches durch die Pumpe P 4 nach B 5 befördert wird, hinzugemischt. Damit kann eine genaue Antikoagulation Milliliter/Zeiteinheit dem definierten Produkt in B 5 zugeführt werden.

12. Technische Neuerung - Veränderung 4 (Skizze 1 Seite 45)

Die technische Veränderung 4 ist durch das Anschweißen eines an den Behälter **B 2** konnektierbaren Schlauches (Detailskizze Anhang Seite 46 u. 47) über eine „Y-Verbindung" (out-line) gekennzeichnet. Während des Primärspülvorganges ist das outlet-Ventil (Rasterklemme) durchgehend geöffnet. Dieses Ventil wird während des sekundären Spülvorganges nach den Erläuterungen auf Seite 32-33 geschlossen. Über diese zusätzliche Linie wird der notwendige geschlossene Kreislauf während der Primärspülung geschaffen.

13. Technische Verbesserung der Sammelpumpe P4 (Skizze 1 Anhang Seite 45)

Die technische Veränderung der Sammelpumpe P 4 ist dadurch gekenn-zeichnet, daß diese Pumpe durch einen Riemenantrieb bewegt wird (Detailskizze Anhang Seite 50). Um eine möglichst genaue Sammlung (0,1 ml/min Intervalle) über den Schlauch, welcher aus der Zentrifugenkammer über P 4 nach B 5 (Sammelbeutel) führt, zu erreichen, darf P 4 keine ruckartigen Bewegungen durchführen. Das Getriebe von P 4 muß über sehr kleine Radbewegungen verfügen können um auch extrem kleine Volumen-Zeit-Einheiten, gemessen am Sammelschlauchdurchmesser zu transportieren. Gegenwärtig sind die eingesetzten Pumpen mit dieser Technik nicht ausgestattet. Ruckartige oder diskontinuierliche Bewegungen bewirken, daß im Sammelschlauch Sogwirkungen entstehen, welche eine übermäßige Kontamination mit unerwünschten Zellkomponenten in B 5 zur Folge haben und somit die Qualität des Produktes verschlechtern. Ferner treten durch solche Sog-wirkungen Veränderungen des **TrPVat$_{ml/min}$** ein, welches eine Unterbrechung des Sammelvorganges zur Folge hat. P 4 wird über ein Zahnradgetriebe, welches ein Rad an dem der Riemen, welcher die Verbindung zur Pumpe P 4 herstellt, angetrieben. Damit können ruckartige Bewegungen vermieden und kleinere Volumen pro Zeiteinheit transportiert werden.

14. Im nachfolgenden wird dargelegt, daß für eine spezifische Sammlung entsprechender weißer Blutkörperchen, Lymphozyten, Blutstammzellen und Monozyten, sowohl während der Zellapherese als auch in einer nachgeschalteten Prozedur, spezifische, auf die jeweilige Zellkomponente ausgerichtete Sedimentationsbeschleuniger eingesetzt werden könnten. Diese müßten über die in Tabelle 9a +9b angezeigten Näherungswerte verfügen.

Tabelle 9a

|  | **Dichte** | $\nu_{sp}$ | **K** | $\chi$ |
|---|---|---|---|---|
| Für | g/ml | cm$^3$g$^{-1}$ | Nm$^{-2}$ | Nm$^{-2}$ |
| Lympho | 1,0587 | 0,94457 | 1,90784E+09 | 5,24153E-10 |
| CD34+ | 1,0626 | 0,94106 | 1,90082E+09 | 5,26088E-10 |
| Mono | 1,0846 | 0,92207 | 1,8623E+09 | 5,3697E-10 |

Tabelle 9b

|  | $f_R$ | $D_K$ | **RT** |
|---|---|---|---|
| Für | Nscm$^{-1}$ | cm$^2$s$^{-1}$ | J/mol |
| Lympho | 1,750E-05 | 2,347E-11 | 2,4611E+01 |
| CD34+ | 2,159E-05 | 1,902E-11 | 2,4611E+01 |
| Mono | 1,759E-05 | 1,865E-11 | 2,4611E+01 |

Die spezifischen Sedimentationsbeschleuniger würden dann als Makromoleküle in Abhängigkeit von der zugeführten Konzentration, zum Einen, die entsprechende Zellkomponenten welche die größere Sediment- ationskonstante besitzen in ihrer Sedimentation beschleunigen. Zum Anderen eine Grenzschicht zwischen diesen und der definierten Zellkomponente, welche gesammelt werden soll bilden, und damit die qualitative Sammlung weiter verbessern.

15. In der Tabelle 10 wird dargelegt, welche Ergebnisse z.B. bei der Sammlung von Blutstammzellen erreicht werden, wenn der neu berechnete Trennungsoptimierungskoeffizient = T$_{OK}$ angewendet wird. Dadurch wird der Thrombozytenverlust des Spenders im Verhältnis zu den bisher verwendeten T$_{OK}$'s im Mittel um 70%, die benötigte Menge Antikoagulanz, welche der Spender erhält, um 66% reduziert.

Tabelle 10

| | N | Minimum | Maximum | Mean | Std. Deviation |
|---|---|---|---|---|---|
| Blutvolumen | 155 | 3469 | 6900 | 4823,89 | 860,98 |
| Proz.-Volumen | 155 | 3033 | 39025 | 24119,92 | 7073,04 |
| Proz.-Zeit-Min | 155 | 50 | 282 | 180,81 | 39,39 |
| ACD-Verbrauch | 155 | 77 | 1380 | 650,55 | 201,14 |
| Sammel-Volumen | 158 | 50 | 400 | 224,35 | 66,06 |
| Thrombo-VorApherese | 150 | 1,6E+02 | 6,8E+05 | 1,5E+05 | 1,0E+05 |
| Thrombo-Nach-Apherese | 126 | 1,2E+04 | 5,6E+05 | 1,4E+05 | 9,4E+04 |
| Thrombo-Verlust | 151 | -2,2E+04 | 3,2E+05 | 3,6E+04 | 5,9E+04 |
| Thrombo-Verl.-Proz * 100 | 115 | ,0100 | ,1830 | ,107202 | 8,45E-02 |
| Leuko-Kons.-Absolut | 158 | ,0000 | 2,8E+11 | 2,7E+10 | 2,7E+10 |
| MNC-Konz.-Absolut | 156 | ,0000 | 2,1E+11 | 1,7E+10 | 1,9E+10 |
| MNC-Konz.-Proz. | 139 | 75 | 100 | 91,71 | 7,94 |
| CD34+/kg | 110 | 0,0E+00 | 2,7E+07 | 3,2E+06 | 4,5E+06 |
| Thrombo-Konz./100 ml | 156 | ,0000 | 2,4E+11 | 3,0E+10 | 3,7E+10 |
| Thrombo-Konz.-Absolut | 156 | ,0000 | 3,8E+11 | 6,3E+10 | 7,2E+10 |
| | N | Minimum | Maximum | Mean | Std. Deviation |
| Hämatokrit | 141 | ,000 | ,130 | 5,43E-02 | 1,89E-02 |
| Ery.-Konz.-Absolut | 156 | ,0000 | 4,3E+11 | 1,1E+11 | 7,6E+10 |

16. Für die spezifische Sammlung spezifischer weißer Blutkörperchen, namentlich von Lymphozyten, Monozyten und ProgentiorBlutstammstellen werden bekanntermaßen spezifische Aufreinigungsfahren, Purging-Verfahren, eingesetzt. Bei diesen Verfahren stören insbesondere die Monozyten in den Leukapherese-Produkten, da diese insbesondere viele Dynabeads binden und somit für das eigentliche Purging nicht mehr zu Verfügung stehen. Im nachfolgenden wird dargelegt, daß das Apherese-Produkt über einen Schlauch (Einlaßseite) mit einem aus Silikon beschichteten Zylinder oder einen aus Glas bestehenden Zylinder, mit einer Oberfläche von > 600 cm$^2$, eingefüllt wird. Die jeweiligen Endseiten sind mit dem gleichen Material ausgestattet und verfügen über einen Leuer-Anschluß. Dieser Zylinder befindet sich auf einem um 360 Grad beweglichen Stativ 2 Umdrehung pro Minute, wobei innerhalb des Stativs der Zylinder ebenfalls im 360 Grad mit einer Geschwindigkeit von 4 Umdrehungen pro Minute bewegt wird. Das Apherese-Produkt wird dadurch gut suspendiert, so daß die Monozyten auf der Oberfläche adhärieren können. Die Inkubationszeit beträgt 30 Minuten. Am Ende der Prozedur wird das Produkt über einen konnektieren Schlauch (Auslaßseite) in einen entsprechenden Beutel überführt und kann dem Purging-Verfahren zugeführt werden.

17. Im nachfolgenden wird dargelegt, daß die unter Punkt 16 an die Zylinder-Oberfläche adhärierten Monozyten mit einer 5% Humanalbumin-Lösung resuspendiert werden. Diese werden dann zur weiteren Kultivierung von den-dritischen Zellen verwendet.

## Anhang

$n$ = Anzahldichte (z.B. Thrombozyten 300 * $10^3/\mu l$)

$_0$ = Anzahldichte bei Start $\Rightarrow n_0$

$_r$ = Anzahldichte bei definierten Umdrehungsgeschwindigkeiten der Zentrifuge $\Rightarrow n_r$

$M$ = Masse des Makromoleküls $M_{molar}$

$m = M_{molar}/ N_A \Rightarrow M_{molar} = m * N_A$

$N_A$ = Avogadro-Zahl 6,0220921 * $10^{23}$ $mol^{-1}$

$k$ = Boltzmann-Konstante 1,380658 * $10^{-23}$ J/K (K = Kelvin)

$T$ = Absolute Temperatur Kelvin ($t_c$/($^0$C + 273) (abs. Nullpunkt = -273 K)

$D$ = Diffusionskoeffizient $\Rightarrow$    $D = kT/f$

$f_R$ = Reibungskoeffizient $\Rightarrow$    $f_R = 6 \pi r \eta$

$r_K$ = Radius des Elementes (z.B. Thrombozyten = 1,38 * $10^{-06}$ m)

$\eta$ = dynamische Viskosität des strömenden Mittels

für Plasma   1,73 * $10^{-3}$ Ns $m^{-2}$ (bei 23$^0$C bzw. 296 K)

[von 23$^0$C bis 30$^0$C 0,032857142 $10^{-3}$ Ns $m^{-2}/^0$C]

für Plasma   **1,50 * $10^{-3}$ Ns $m^{-2}$** (bei **30$^0$C** bzw. 306 K)

[von 30$^0$C bis 37$^0$C 0,028571428 $10^{-3}$ Ns $m^{-2}/^0$C]

für Plasma   1,30 * $10^{-3}$ Ns $m^{-2}$ (bei 37$^0$C bzw. 313 K)

für Serum   0,94 * $10^{-3}$ Ns $m^{-2}$ (bei 23$^0$C bzw. 296 K)

[von 23$^0$C bis 30$^0$C 0,02 $10^{-3}$ Ns $m^{-2}/^0$C]

für Serum   **0,80 * $10^{-3}$ Ns $m^{-2}$** (bei **30$^0$C** bzw. 306 K)

[von 30$^0$C bis 37$^0$C 0,015 $10^{-3}$ Ns $m^{-2}/^0$C]

für Serum   0,695 * $10^{-3}$ Ns $m^{-2}$ (bei 37$^0$C bzw. 313 K)

für Blut     4,00 * $10^{-3}$ Ns $m^{-2}$ (bei 23$^0$C bzw. 296 K)

$\sigma_K$ = Dichte des Körpers (z.B. Thrombozyt 1,040 kg $dcm^{-3}$)

$\sigma_{fl}$ = Dichte des strömenden Mediums (z.B. Plasma 1,030 kg $dcm^{-3}$)

$v$ = Geschwindigkeit des Elementes (m/s)

$Fv_K$ = Füllvolumen des Zentrifugenkanals

$V$ = Volumen des Körpers bzw. Elementes (dcm * $10^3$ = Liter)

$m$ = Masse des Elementes bzw. Körpers ($m = V * \sigma$ = kg)

$g$ = Erdbeschleunigung = 9,81 m/s

$G$ = Gravitationskonstante 6,6726 * $10^{-12}$ $m^3$ $kg^{-1}$ $s^{-2}$

$r_z$ = $r_{max}$ Abstand vom Zentrum der Rotation

$r_{zStA(Ery)}$ = Abstand vom Zentrum der Rotation Außwand des Kanals

$r_{zStM(Ery)}$ = Abstand vom Zentrum der Rotation zur Mittelwand des Kanals

$r_{zStI(Ery)}$ = Abstand vom Zentrum der Rotation zur Innenwand des Kanals

$r_{WBCA}$ = Abstand vom Zentrum der Rotation zur Außenwand des Kanals

$r_{WBCK}$ = Abstand vom Zentrum der Rotation des Konus im Kanal

$r_{WBCKI}$ = Abstand vom Zentrum der Rotation zur Innenwand des Kanals

$r_{TkrPIA}$ = Abstand vom Zentrum der Rotation zur Außenwand des Kanals

$r_{TkrPII}$ = Abstand vom Zentrum der Rotation zur Innenwand des Kanals

Ery = Erythrozyt

WBC = white blood component (z.B. Monozyten oder Blutstammstellen)

TkrPl = Thrombozytenreiches Plasma

Tk = Thrombozyt

$a_r$ = Zentripetalbeschleunigung

$a_r$ = $r * \omega^2 = r * (2\pi f)^2$

$\omega$ = Winkelgeschwindigkeit = $2\pi f$ ($f$ = Umlauffreqenz =

Umdrehungszahl/60) [$s^{-1}$]

$a_z$ = Zentripetalkraft $\Rightarrow$ $a_z = m * r * \omega^2 = m * r * (2\pi f)^2$

RT= $N_A * k * T$

kT= thermische Konstante J K

$p$ = Druck = 1 $N/m^2$ = 1 Pascal = $10^{-5}$ bar (1 Newton = kg $m/s^2$) $\Rightarrow$

1 bar = $10^5$ Pa

1 at = 0,980665 bar

1 atm = 1013,25 mbar

$\Rightarrow$

1 $N/m^2$ = 1,02 * $10^{-5}$ at

1 $N/m^2$ = 9,87 * $10^{-6}$ atm

## Graphik 1

rpm Blutstammzellsammlung

## Graphik 2

rpm Thrombozytensammlung

In den Graphiken 1 und 2 ist der Unterschied zwischen den aktuellen Umdrehungsgeschwindigkeiten, welche für alle Zuflußvolumen gleich sind und den neu berechneten dargestellt.

In den Graphik 3 und 4 sind in Abhängigkeit von den Einlaßflüssen, dem Hämatokrit und der Anzahldichte der Komponenten, die Volumen pro Minute der Pumpen P 3 bzw. P 4 dargestellt.

Graphik 3

TrPVat (Thrombozytenreicher Plasmaüberstand) Blutstammzellsammlung in Abhängigkeit vom Bluteinlaß ml/min

ml

120

90

60

30

0

20 21 22 23 24 25 26 27 28 29 30 31 32 33 34 35 36 37 38 39 40 41 42 43 44 45 46 47 48 49

Hämatokrit

Graphik 4

Sammelflußvolumen Blutstammzellsammlung in Abhängigkeit vom Hämatokrit

Einlaß ml/min

ml/min

2,5

0,0

Fluß – Diagramm 1
Sequentielles Verfahren

Fluß – Diagramm 2
Kombiniertes Verfahren

Skizze 1

Technische Veränderung 1    Technische Veränderung 2    Technische Veränderung 3

Technische Veränderung 4

B1  B2  B3  B4  B5

P5

P1  P2  P3  P4

Technische Verbesserung

MK1    MK2

Outlet-Line

Inlet-Line

Zentrifugenkammer

Zentrifuge

EP 0 976 414 A2

## Detailskizzen für die zusätzlichen Schläuche und Beutelanschlüsse

Zu Punkt 12

Die technische Veränderung 4, ist gekennzeichnet durch eine Schlauchverbindung für die Entlüftung über den Rückflußschlauch zu B 1 bzw. B 2
über MK 2, wie im nebenstehenden Kasten (Neu) dargestellt.

Nach B 1 (NaCl 0,9%) von MK2

Teil A/B

Teil C

| Nach B 2 (Humanalbumin 5%) von MK2 |
| Neu          Teil B/A |
| Teil C |
| Verbindungsstelle |

⇧

Von MK2

Anstecknadel zur Lösungsrückführung (Teil B) und Tropfkammer (Teil A)

Schlauch

Teil B          Teil A

28

Rasterkemme (Teil C)

Schauch

Zu Punkt 9

Die technische Veränderung 1, ist gekennzeichnet durch eine Schlauch-verbindung zu B1 bzw. neuen B 2, wie im nebenstehenden Kasten (Neu) dargestellt.

Von B 1 (NaCl 0,9%) nach

P1Von B 2 (Humanalbumin 5%) nach P1

**Neu** / Teil E/D

Teil F

Verbindungsstelle

Nach P1

Rollklemme (Teil F)

Schlauch

Ansteckverbindung zur Lösungsentnahme (Teil D) und Tropfkammer (Teil E)

Schlauch

Teil E      Teil D

Zu Punkt 10

Die technische Veränderung 2, ist gekennzeichnet durch eine Schlauch-verbindung zu B 5 und zur Perfusorpumpe, wie im nebenstehenden Kasten (Neu) dargestellt.

Nach B 5 durch Sammelschlauch

Von P 5 durch ACD-Perfusorschlauch

**Neu**          Teil G

Teil D

Verbindungsstelle

Von P5

30

Leuer-Anschluß (Teil G) am Ende von Schlauch A zur Konnektierung von z.B. Perfusorspritze

Verschlußkappe

Teil G

# Detailskizze kennzeichnet Riemenantrieb der Pumpe P 4 (=Sammelpumpe)

Sammelpumpe P4

Sammelfluß

Riemenlaufwalze

Übertragungsrad

Riemen

Verbindungsstange zum Sammelpumpenrad

Zahnradbewegungs-übertragungsverbindung (Stange)

Riemenlaufwalze

Motor für Zahnradantrieb

EP 0 976 414 A2

Zentrifugalkraft in Abhängigkeit vom Drehachsenabstand der Zentrifuge

Tabelle 11

| az A1 | az A2 | az A3 | az A4 |
|---|---|---|---|
| Zentrifugalkraft | Zentrifugalkraft | Zentrifugalkraft | Zentrifugalkraft |
| 100 | 99 | 102 | 96 |
| 150 | 148 | 153 | 144 |
| 199 | 196 | 203 | 191 |
| 249 | 246 | 254 | 238 |
| 299 | 295 | 305 | 286 |
| 350 | 344 | 356 | 334 |
| 399 | 393 | 406 | 382 |
| 450 | 444 | 458 | 430 |
| 499 | 492 | 508 | 477 |
| 549 | 541 | 559 | 525 |
| 599 | 590 | 610 | 573 |
| 649 | 640 | 661 | 621 |
| 700 | 690 | 713 | 669 |
| 750 | 739 | 764 | 717 |
| 800 | 788 | 814 | 765 |
| 849 | 836 | 864 | 811 |
| 899 | 886 | 915 | 860 |
| 949 | 935 | 966 | 907 |
| 999 | 985 | 1018 | 956 |

| az A1 | az A2 | az A3 | az A4 |
|---|---|---|---|
| Zentrifugalkraft | Zentrifugalkraft | Zentrifugalkraft | Zentrifugalkraft |
| 1049 | 1034 | 1068 | 1003 |
| 1100 | 1084 | 1120 | 1052 |
| 1149 | 1133 | 1170 | 1099 |
| 1200 | 1182 | 1222 | 1147 |
| 1249 | 1230 | 1271 | 1194 |
| 1299 | 1280 | 1322 | 1242 |
| 1349 | 1330 | 1374 | 1290 |
| 1398 | 1378 | 1424 | 1337 |
| 1448 | 1427 | 1474 | 1385 |
| 1499 | 1477 | 1526 | 1433 |
| 1547 | 1524 | 1575 | 1479 |
| 1599 | 1576 | 1628 | 1529 |
| 1649 | 1625 | 1679 | 1577 |
| 1697 | 1672 | 1728 | 1623 |

**Patentansprüche**

1. Verfahren zur Sammlung von spezifischen Blutkomponenten bei kontinuierlichen, laminaren Strömungsverhältnissen, wobei dem Patienten mittels einer ersten Leitung Blut entnommen und einer der Separation der Blutkomponenten dienenden Zentrifuge zugeführt und über eine Rückführleitung dem Patienten Blut mit der Entnahme-Zuflußrate zurückgeführt wird, wobei die Separation bestimmt wird durch den Geometriefaktor der Zentrifuge, durch die Rotationsgeschwindigkeit der Zentrifuge und die Positionierung der Entnahmeleitung an der Zentrifuge, und wobei charakteristisch für die Separation einzelner bestimmter Blutkomponenten ein Trennungsoptimierungskoeffizient $T_{OK}$ ist, **dadurch gekennzeichnet, daß** zur Sammlung von Blutstammzellen bzw. mononuklearen Zellen der Trennungsoptimierungskoeffizient $T_{OK}$ zwischen 140 und 190, vorzugsweise bei ca. 176 liegt, und/oder daß bei Sammlung von Thrombozyten bei Blutflußgeschwindigkeiten zwischen 30 und 100 ml/min der Trennungsoptimierungskoeffizient zwischen 600 und 700, vorzugsweise bei ca. 649 liegt.

2. Verfahren nach Anspruch 1, wobei vor der Blutentnahme von einem Patienten das Schlauchsystem mit einer in einem ersten Behälter B1 bevorrateten physiologischen Kochsalzlösung gespült wird und aus dem Schlauchsystem zu entfernende Luft in einen Behälter B4 gepumpt wird, und wobei bei Beginn der Blutentnahme über eine erste Pumpe P1 das Blut des Patienten entnommen und in einer Mischkammer MK1 in einem definierten Verhältnis mit einem Antikoagulanz versetzt wird, **dadurch gekennzeichnet, daß** ein zusätzlicher Behälter B2 mit Humanalbumin-Lösung parallel geschaltet zum Behälter B1 mit physiologischer Kochsalzlösung angeordnet wird, wobei zunächst eine Primärspülung mit diesem Humanalbumin und anschließend in an sich bekannter Weise eine Spülung mit physiologischer Kochsalzlösung vorgenommen wird, wobei der Großteil der Lösung der Primärspülung entfernt wird.

3. Verfahren nach Anspruch 2, **dadurch gekennzeichnet, daß** das für die Primärspülung verwendete Humanalbumin in den Behälter B2 zurückgepumpt wird.

4. Verfahren, insbesondere nach Anspruch 1 oder 2, wobei die separierten definierten Blutkomponenten aus der Kammer der Zentrifuge über eine Pumpe P4 abgepumpt und einem Sammelbehälter zugeführt werden, **dadurch gekennzeichnet, daß** der Pumpe P4 eine Abzweigung nachgeordnet ist, die einerseits in den Sammelbehälter B5 für die separierten Blutkomponenten führt und in die andererseits die von einer zusätzlichen Pumpe P5 kommende Leitung einmündet, wobei über die Pumpe P5 während des Sammelvorgangs ein Antikoagulanz zugeführt wird, wobei gleichzeitig die dem Patienten zugeführte Menge an Antikoagulanz reduziert wird.

5. Verfahren nach Anspruch 4, **dadurch gekennzeichnet, daß** die Dosierung des über die Pumpe P5 zugeführten Antikoagulanz in Abhängigkeit von dem Sammelvolumen zugeführt wird, welches von der Pumpe P4 aus der Zentrifugenkammer zum Sammelbehälter B5 gepumpt wird.

6. Vorrichtung zur Durchführung des Verfahrens nach einem der Ansprüche 1 bis 5, umfassend eine Pumpe P4 zum Abpumpen der separierten Blutkomponenten aus der Zentrifugenkammer zu einem Sammelbehälter B5, **dadurch gekennzeichnet, daß** die Pumpe P4 über einen Riemenantrieb angetrieben ist.